# EUROPEAN PATENT APPLICATION

(11) **EP 2 333 058 A1**
(43) Date of publication of application: **15.06.2011**
(21) Application number: 09806736.6
(22) Date of filing: 12.08.2009
(51) Int. Cl.: C12N 15/00, A01G 7/06, A01H 1/00, A01N 25/00, A01N 63/00, A01P 21/00, C12N 15/09, C07K 14/195

(54) **METHOD FOR PROMOTION OF BRANCH FORMATION, DWARFING, STERILITY AND PROMOTION OF MULTIPLE FLOWER BUD FORMATION OF PLANT**

(30) Priority: 12.08.2008 JP 2008208152
(71) Applicant: The University of Tokyo, Bunkyo-Ku Tokyo 113-8654 (JP)
(72) Inventor: NAMBA, Shigetou, Tokyo 113-8654 (JP); OSHIMA, Kenro, Tokyo 113-8654 (JP)
(74) Representative: Creek, Isobel Clare
(86) International application number: PCT/JP2009/064252
(87) International publication number: WO 2010/018845

(57) **Abstract**

An object of the present invention is to provide a method for promoting branch formation, inducing dwarfism, causing sterility, and promoting multiple flower bud formation, of a plant, and an agent for promoting branch formation and/or inducing dwarfism and/or causing sterility and/or promoting multiple flower bud formation, of a plant. Phytoplasmas are pathogenic bacteria that promote branch formation and/or dwarfism and/or sterility and/or the multiple flower bud formation of a plant. Introduction of a PAM765 peptide derived from a phytoplasma into a plant enables the promotion of branch formation and/or dwarfism and/or sterility and/or the promotion of multiple flower bud formation of a plant. It is also possible to provide an agent for promoting branch formation and/or inducing dwarfism and/or causing sterility and/or promoting multiple flower bud formation of a plant, the agent containing the PAM765 peptide or a gene encoding the PAM765 peptide.

## Description

### Cross Reference to Related Application

This application claims the benefit of priority from Japanese Patent Application No. 2008-208152 filed August 12, 2008, which is herein incorporated by reference.

### Technical Field

The present invention relates to methods for promoting branch formation, inducing dwarfism, causing sterility, and promoting multiple flower bud formation of plants.

### Background Art

If adjustment of growth and control of morphogenesis in plants can be realized, the yield of agricultural crops can be increased by, for example, improving photosynthesis efficiency or increasing the number of ears. Furthermore, as for garden plants, a significant improvement of value added can be expected by controlling morphological characteristics. Hitherto, in general, mutation of a series of morphogenesis-related genes of plants has been induced by treating them with a mutagen or the like, and useful mutants are selected from the induced mutants and used for molecular bleeding. In this method, however, influences of the introduction of the trait on other various traits such as the quality and growth characteristics are unknown, and it is difficult to predict whether the mutant genes can become useful and practical or not.

*Phytoplasma asteris* is a bacterium pathogenic to plant, which infects 700 types of plants or more and causes characteristic morphological changes (refer to, for example, Lee IM et al., Annual Review of Microbiology 54: 221-255 (2000)). Examples of morphological abnormalities observed in plants by such infection include dwarfism, an increase in branch formation (witches'-broom), yellowing, curly leaf, transformation of floral organs to leaves (phyllody), and transformation of floral organs to shoot structures (proliferation). These morphological abnormalities are serious diseases in agriculture and cause devastating damage. On the other hand, a hydrangea that exhibits a phyllody symptom, a poinsettia that exhibits a witches'-broom, and the like are also known as plants exhibiting disease symptoms caused by infection with a phytoplasma (refer to, for example, Lee IM et al., Nature Biotechnology 15: 178-182 (1997)). However, these morphologically abnormal plants increase the commercial values of the plants because of the uniqueness of their morphology, and such breeds have been registered and are highly valued.

Accordingly, it is believed that growth and morphology of plant individuals are controlled by using the infection with a phytoplasma which is a pathogenic bacterium, whereby useful traits such as branch formation and dwarfism can be provided to the plant individuals. However, since the infection with phytoplasmas usually spreads through leafhoppers which are insect vectors (refer to, for example, Suzuki S et al., Proceedings of the National Academy of Sciences 103: 4252-4257 (2006)), even if plant individuals infected with a phytoplasma have a useful trait, the plant individuals may become a source of generation of a new disease in other plant individuals.

### Summary of Invention

### Technical Problem to Be Solved

Accordingly, it is an object of the present invention to provide a method for promoting branch formation, inducing dwarfism, causing sterility, and promoting multiple flower bud formation of plants without mediating infection with a phytoplasma, and an agent for promoting branch formation, inducing dwarfism, causing sterility, and promoting multiple flower bud formation of plants without mediating infection with a phytoplasma.

### Solution to Problem

The inventors of the present invention conducted screening of peptide candidates that promote branch formation and dwarfism in plants by expressing all genes that are assumed to be encoded by DNA of *Phytoplasma asteris* in plant individuals one by one and observing change in the morphology of the individuals. As a result, the inventors of the present invention found a PAM765 peptide (TENGU) that promotes morphological abnormalities such as increase of branching and dwarfism, and this finding led to the completion of the present invention.

Herein, the term "branching" refers to a branch formed by the growth of a lateral bud generated from a node of a stem forming a main axis of a plant individual. This branch may have a leaf and a flower in addition to a stem structure with its growth. The term "multiple flower bud formation" means that multiple flower buds are formed from one node of a stem of a plant without branching, and the term "flower bud formation" includes processes of initiation, differentiation, and development of flower buds.

Specifically, a method for promoting branch formation, and/or inducing dwarfism, and/or causing sterility, and/or promoting multiple flower bud formation of a plant according to the present invention includes introducing a peptide having a PAM765 peptide into the plant without infection with a non-recombinant phytoplasma. The PAM765 peptide has preferably any one of amino-acid sequences represented by DQDDDIENVITLX₄ETKENQTEX₅IKX₆QCQDLLQKGEKDA (Seq No. 1), DQDDDIENVITLIETKENQTEQIKIQCQDLLQKGEKDA (Seq No. 2), and DQDDDIENVITLTETKENQTEEIKMQCQDLLQKGEKDA (Seq No. 3). The PAM765 peptide may have an amino-acid sequence in which several amino-acid residues are deleted, inserted, or substituted in Seq No. 1 to 3, and can promote branch formation, and/or inducing dwarfism, and/or causing sterility of a plant. (Note that, in the above sequences, X₄ to 6 may be any amino acid.)

In the method for promoting branch formation, and/or inducing dwarfism, and/or causing sterility, and/or promoting multiple flower bud formation of a plant according to the present invention, DNA encoding the PAM765 peptide may be expressed in the plant. The expression of the DNA in the plant may be performed by producing a transgenic plant.

An agricultural-chemical composition or an agent for promoting branch formation of a plant, and/or inducing dwarfism, and/or causing sterility, and/or promoting multiple flower bud formation of a plant according to the present invention contains a PAM765 peptide or a gene encoding the PAM765 peptide.

### Brief Description of Drawings

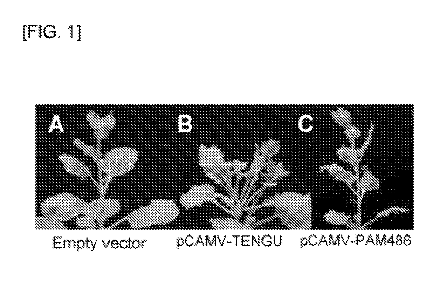

[Fig. 1] Fig. 1 includes photographs each showing a tobacco plant into which an empty pCAMV vector (control group, A), a pCAMV vector into which DNA encoding a PAM765oy peptide was inserted (pCAMV-TENGU, B), or a pCAMV vector into which PAM486 was inserted (control group, C) was introduced via Agrobacterium in an example of the present invention.

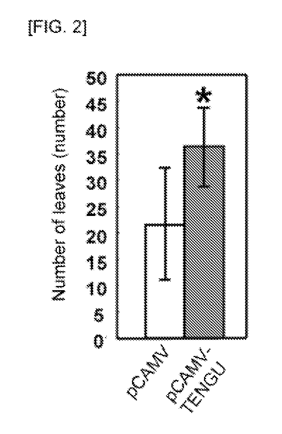

[Fig. 2] Fig. 2 is a graph showing the number of leaves of tobacco plants in which the empty pCAMV vector (control group, A) or the pCAMV vector into which the DNA encoding the PAM765oy peptide was inserted (pCAMV-TENGU, B) was introduced via Agrobacterium in an example of the present invention. Note that the data is represented by average + standard deviation (*p < 0.05)(N=4 (pCAMV), N=8 (pCAMV-TENGU)).

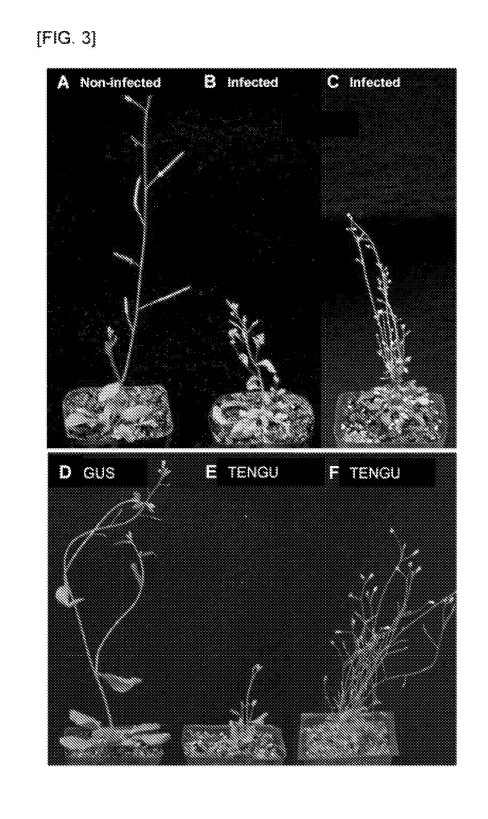

[Fig. 3] Fig. 3 includes photographs of phytoplasma uninfected wild-type (A), infected via leafhopper (B, C), GUS transgenic (D), and Tengu transgenic (E) *Arabidopsis* thaliana plants in an example of the present invention.

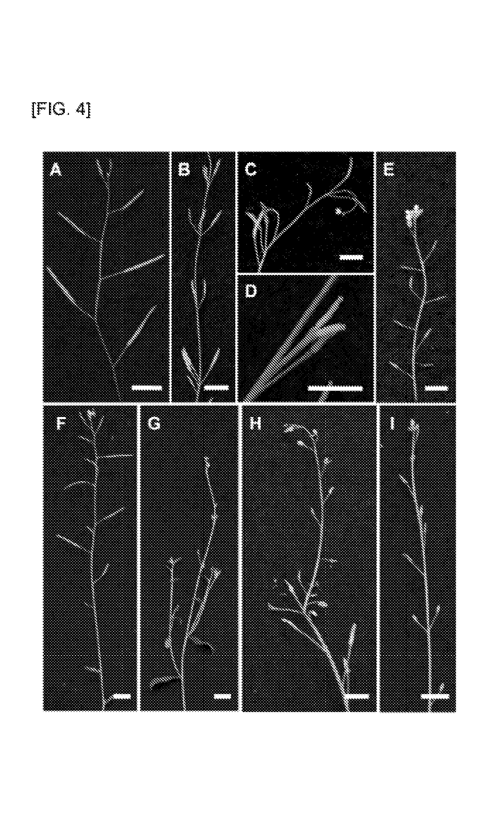

[Fig. 4] Fig. 4 includes photographs of GUS transgenic (A), infected via leafhopper (B to E), phytoplasma uninfected wild-type (F), and Tengu transgenic (G to H) *Arabidopsis* thaliana plants in an example of the present invention. The scale bar represents 50 mm.

### Description of Embodiments

Embodiments of the present invention completed on the basis of the above finding will now be described in detail by way of Examples. However, the present invention is not limited to Examples below.

Methods described in standard protocols such as J. Sambrook, E. F. Fritsch & T. Maniatis (Ed.), Molecular cloning, a laboratory manual (3rd edition), Cold Spring Harbor Press, Cold Spring Harbor, New York (2001); F. M. Ausubel, R. Brent, R. E. Kingston, D. D. Moore, J. G. Seidman, J. A. Smith, K. Struhl (Ed.), Current Protocols in Molecular Biology, John Wiley & Sons Ltd., or methods obtained by modifying or altering the above methods are used unless otherwise specifically described in embodiments and Examples below. When a commercially available reagent kit or measuring device is used, a protocol attached thereto is used unless otherwise specifically described.

It should be understood that objects, features, advantages, and ideas of the present invention are apparent to those skilled in the art, and those skilled in the art can easily implement the present invention on the basis of the description of this specification. Embodiments of the present invention, specific Examples etc. described below show preferred embodiments of the present invention, and are illustrative and explanatory, and the present invention is not limited thereto. It should be understood that various modifications can be made by those skilled in the art on the basis of the description of this specification within the aim and scope of the present invention disclosed herein.

### <1. Method for promoting branch formation, inducing dwarfism, causing sterility, and promoting multiple flower bud formation of plants >

A method for promoting branch formation, inducing dwarfism, causing sterility, and promoting multiple flower bud formation of plants according to the present invention includes introducing a PAM765 peptide into a plant individual without infection by a non-recombinant phytoplasma.

Herein, the non-recombinant phytoplasma refers to a phytoplasma other than phytoplasmas produced by using a gene recombination technique. Examples thereof include wild-type phytoplasmas and phytoplasmas having a spontaneous mutation.

The PAM765 peptide represents peptides having a length of 70 amino-acid residues at a maximum, and represents the following peptides:
(1) peptides having amino-acid sequences of Seq No. 4 to 6 below:
   MVKLX₁KX₂KX₃KLLIFAGFWAILLFLNHNYLIFADQDDDIENVITLX₄ETKENQTEX₅IKX ₆QCQDLLQKGEKDA (Seq No. 4) (wherein X_{1 to 6} may be any amino acid, however, preferably, X₁ is K or Q, X₂ is H or D, X₃ is A or V, X₄ is I or T, X₅ is Q or E, and X₆ is I or M),
   MVKLKKHKAKLLIFAGFWAILLFLNHNYLIFADQDDDIENVITLIETKENQTEQIKIQCQ DLLQKGEKDA (Seq No. 5), and
   MVKLQKDKVKLLIFAGFWAILLFLNHNYLIFADQDDDIENVITLTETKENQTEEIKMQCQ DLLQKGEKDA (Seq No. 6);
(2) peptides having amino-acid sequences of Seq No. 1 to 3 below:
   DQDDDIENVITLX₄ETKENQTEX₅IKX₆QCQDLLQKGEKDA (Seq No. 1) (wherein X_{4 to 6} may be any amino acid, however, preferably,
   X₄ is I or T, X₅ is Q or E, and X₆ is I or M),
   DQDDDIENVITLIETKENQTEQIKIQCQDLLQKGEKDA (Seq No. 2), and
   DQDDDIENVITLTETKENQTEEIKMQCQDLLQKGEKDA (Seq No. 3);
(3) peptides having a partial peptide composed of an amino-acid sequence corresponding to the amino-acid sequence of Seq No. 2 among peptides encoded by homologs and orthologs of a gene that encodes the peptide composed of the amino-acid sequence of Seq No. 5,;
(4) peptides that are composed of a part of an amino-acid sequence of any one of Seq No. 4 to 6 and that has an amino-acid sequence of any one of Seq No. 1 to 3 or the amino-acid sequence of Seq No. 2 described in (3), the amino-acid sequence corresponding to the amino-acid sequence represented by sequence number 2; and
(5) mutant peptides having an amino-acid sequence in which, in the sequence of a peptide described in any one of (1) to (4), several amino-acid residues (for example, 10 amino-acid residues, preferably, 8 amino-acid residues, more preferably, 6 amino-acid residues, still more preferably, 4 amino-acid residues, and particularly preferably, 2 amino-acid residues) are deleted, inserted, or substituted, and capable of promoting branch formation and/or inducing dwarfism, and/or causing sterility of plants. The following mutant peptides (Seq No. 7 to 9) having methionine added at their N-terminal are preferable.
   MDQDDDIENVITLX₁ETKENQTEx₂IKX₃QCQDLLQKGEKDA (Seq No. 7)
   MDQDDDIENVITLIETKENQTEQIKIQCQDLLQKGEKDA (Seq No. 8)
   MDQDDDIENVITLTETKENQTEEIKMQCQDLLQKGEKDA (Seq No. 9)

The mutation in the mutant peptides of (5) does not occur in an amino acid necessary for a branch formation promotion activity, a dwarfism-inducing activity, or a sterility-causing activity in the peptides of (1) to (4), or even if a mutation occurs, the mutation does not lose the branch formation promotion activity, the dwarfism-inducing activity, or the sterility-causing activity in the peptides of (1) to (4). Thus, the mutant peptides of (5) are obviously considered as equivalents of the peptides of (1) to (4) by those skilled in the art. These peptides may be subjected to a modification such as glycosylation.

The target plant to which the method of the present invention can be applied is not particularly limited as long as branch formation, dwarfism, sterility, or multiple flower bud formation can be promoted in the plant by the PAM765 peptides described above. Plants in which the phenotype of a disease is expressed by onion yellows (OY) phytoplasma or Aster yellows witches'-broom (AYWB) phytoplasma are preferable. Specific examples of the plant include *Nicotiana benthamiana, Arabidopsis thaliana*, *Chrysanthemum coronarium,* and *Petunia hybrida.*

As described in Examples, the peptide having the amino-acid sequence represented by Seq No. 2, which is included in OY-phytoplasma, has an activity of promotion of branch formation, dwarfism, sterility, and multiple flower bud formation of plants. On the other hand, the peptide having the amino-acid sequence represented by Seq No. 3 is its homologue in AYWB-phytoplasma, and is believed to have the same activity. In these peptides, amino-acid residues at three positions (corresponding to X_{1 to 3} in Seq No. 1) are not conserved. Accordingly, it is believed that these amino-acid residues are not important in the activity of promotion of branch formation, dwarfism, and sterility in plants. Therefore, it is believed that the peptide having the amino-acid sequence represented by Seq No. 1, in which those amino acids are not specified, also has the activity of promotion of branch formation, dwarfism, sterility, or multiple flower bud formation in plants.

The method for introducing the PAM765 peptide to a plant individual is not particularly limited, and examples of the method to be used include introduction of the PAM765 peptide itself, introduction performed by expressing DNA encoding the PAM765 peptide in the plant individual, and introduction performing by transplanting a cell that expresses the PAM765 peptide. It should be noted that, since the PAM765 peptide has a significantly low molecular weight, when the PAM765 peptide is introduced into a portion of a plant individual, the peptide diffuses to a position where the peptide should function. Accordingly, the PAM765 peptide may be introduced into either a part of the plant individual or the entire part thereof.

### <2. Production of PAM765 peptide and introduction of the PAM765 peptide into plant individual>

As described above, to introduce PAM765 peptide into a plant individual, the PAM765 peptide itself may be introduced into a plant individual.

The method for obtaining or preparing the PAM765 peptide to be introduced into a plant individual is not particularly limited. The PAM765 peptide may be a naturally-occurring peptide, a recombinant peptide produced by using a gene recombination technique, or a peptide that is chemically synthesized by a known method. The naturally-occurring peptide can be obtained from a phytoplasma that expresses the PAM765 peptide by appropriately combining isolation methods and purification methods of protein. Alternatively, the naturally-occurring peptide may be purified from organs of an insect, a plant, or the like infected by a phytoplasma. The strain of the phytoplasma is not particularly limited as long as the phytoplasma promotes branch formation, induces dwarfism, causes sterility, or promotes multiple flower bud formation. When the peptide according to the present invention is prepared by a chemical synthesis, the peptide can be produced in accordance with a chemical synthesis method known to those skilled in the art, such as a fluorenylmethyloxycarbonyl (Fmoc) method or a t-butyloxycarbonyl (tBoc) method. Alternatively, the PAM765 peptide can be produced with a commercially available peptide synthesis device. When the peptide is prepared by using a gene recombination technique, DNA with a nucleotide sequence encoding the PAM765 peptide is inserted into a suitable expression vector, and a host such as a cultured cell is then transformed to express the peptide.

A method for introducing the PAM765 peptide thus obtained into a plant individual is not particularly limited. The peptide may be injected into a portion of the plant individual, such as a leaf, or the vascular bundle with a syringe or the like.

### <3. Introduction of PAM765 peptide into plant individual by expressing DNA encoding PAM765 peptide in plant individual>

As described above, to introduce a PAM765 peptide into a plant individual, DNA encoding the PAM765 peptide may be expressed in the plant individual.

In order to express DNA encoding a PAM765 peptide in a plant individual, first, an expression vector in which the DNA encoding PAM765 is inserted downstream of an appropriate promoter for expressing the DNA in a plant cell is prepared. The PAM765 peptide can be introduced into a plant individual by introducing this expression vector into a living plant, or by introducing this expression vector into a tissue or organ isolated from a living plant, a cell strain derived from a plant, or a callus and then regenerating the tissue or the like to a plant individual,.

In a method for expressing the DNA in a plant individual, first, the DNA encoding the PAM765 peptide is inserted into an expression viral vector by a method known to those skilled in the art to prepare a recombinant viral vector. An example of the viral vector to be used here is pCAMV (potato virus X vector), but is not particularly limited thereto.

The recombinant viral vector thus prepared is introduced into a plant individual. This process can be selected from a method using a living plant and a method using a tissue or organ isolated from a plant, a cell strain derived from a plant, or a callus. These methods will be described in detail below.

First, an example of the method for introducing the recombinant viral vector into a plant using a living plant is an Agrobacterium method in which Agrobacterium is mediated. In the Agrobacterium method, the recombinant viral vector is introduced into Agrobacterium by a method known to those skilled in the art, such as an electroporation method (Nagel et al., Microbiology Letters, 67: 325, 1990).

Next, the resulting recombinant viral vector-introduced Agrobacterium is suspended in an immersion buffer to prepare an Agrobacterium bacterial suspension, and a part of or the entire part of a living plant is immersed in the suspension (Gelvin et al., Molecular Biology Manual, Academic Press Publishers; Cough & Bent, The plant Journal, 16: 735-743, 1996). Thus, the DNA encoding the PAM765 peptide can be introduced into a cell in the immersed part of the living plant, and the PAM765 peptide can be expressed and secreted at the position.

Alternatively, the recombinant viral vector can be directly introduced into a cell of a living plant individual by a method known to those skilled in the art, such as an electroporation method or a particle gun method. Thus, the PAM765 peptide can be expressed in the cell into which the DNA encoding the PAM765 peptide has been introduced.

On the other hand, when the recombinant vector is introduced into a plant individual using cells under culture, such as a tissue, organ, cell strain, or callus derived from a plant, first, the recombinant viral vector is introduced into the cells derived from the plant either via Agrobacterium or directly, thus preparing transformed cells that express the PAM765 peptide.

In Agrobacterium-mediating methods, by co-culturing the cultured-cells with the recombinant viral vector-introduced Agrobacterium to introduce Agrobacterium into the cells, the transformed cells are prepared. In contrast, when the recombinant viral vector is directly introduced into the cells, a method known to those skilled in the art, such as an electroporation method or a particle gun method, may be applied to the cultured-cells.

The resulting transformed cells express the PAM765 peptide. By culturing these transformed cells in accordance with a common procedure in a culture medium containing a phytohormone (such as auxin or cytokinin) for regenerating a plant, the transformed cells can be regenerated to a plant. Thus, a transgenic plant, all the cells of which have the DNA encoding the PAM765 peptide, can be obtained. This DNA expresses the PAM765 peptide under the control of a promoter added upstream of the PAM765 peptide. Alternatively, when a recombinant virus having the DNA encoding the PAM765 peptide is introduced into a part of a plant, the recombinant virus moves to the entire plant, and the plant expresses this peptide throughout the body.

### <4. Agent for promoting branch formation and/or inducing dwarfism and/or causing sterility and/or promoting multiple flower bud formation of plants>

An agricultural-chemical composition for promoting branch formation and/or inducing dwarfism and/or causing sterility and/or promoting multiple flower bud formation of plants according to the present invention contains a PAM765 peptide, a gene encoding the PAM765 peptide, or a combination of two or more of these. The agricultural-chemical composition can be formulated by using additives for formulation known to those skilled in the art. The form of the agricultural-chemical composition is not particularly limited. Examples of the form include an emulsifiable concentrate, a liquid formulation, an oil solution, a watersoluble granule, a wettable powder, a flowable formulation, a dust formulation, a micro granule, a granule, an aerosol, a fumigant, a paste and the like. Other active ingredients of agricultural chemicals such as an insecticide, a germicide, a mixture of an insecticide and a germicide, and an herbicide may be blended in this agent. The method and the amount of use of this agent for promoting branch formation and/or inducing dwarfism and/or causing sterility of plants can be appropriately selected by those skilled in the art in accordance with the conditions such as the purpose of use, the formulation, and the place of use.

### EXAMPLES

### <EXAMPLE 1>

This example shows that a PAM765 peptide of a phytoplasma promotes branch formation and dwarfism in plants.

### == Introduction of a gene encoding PAM765 peptide into a plant individual ==

A DNA sequence (sequence number 10) encoding a PAM765oy peptide (sequence number 8) derived from OY-phytoplasma, which promotes branch formation and dwarfism in plants, was amplified by a polymerase chain reaction (PCR) using KOD DNA polymerase (Toyobo Co., Ltd.). Here, total DNA extracted from a phytoplasma-infected plant by a cetyltrimethylammonium bromide method (Namba et al., Phytopathology, 83: 786-791, 1993) was used as a template. Oligonucleotides having the sequences below were used as PCR primers.
Primer S1:
   acgcgtcgacATGGACCAAGATGATGATATTGAAAACGTGATAACTC (sequence number 11)
Primer AS1:
   tgacccgggTTAGGCATCTTTCTCGCCCTTTTGCAATAAATCTTGACA (sequence number 12)

The PCR product was cleaved with restriction enzymes (SalI and SmaI) and inserted into a multi-cloning site of a binary potato virus X (PVX) vector (pCAMV, which is obtained by inserting cDNA pP2C2S (Baulcombe DC, Chapman S, Santa Cruz S, The Plant Journal 7: 1045-1053, 1995) of PVX into a binary vector pCAMBIA of Agrobacterium) to produce a pCAMV-TENGU recombinant viral vector. This pCAMV-TENGU recombinant viral vector was introduced into Agrobacterium *(A. tumefaciens)* EHA 105 strain by an electroporation method. As control groups, a pCAMV vector that contained no insert DNA, and pCAMV-PAM486 which was a recombinant vector containing DNA encoding a different peptide PAM486 were each introduced into Agrobacterium in the same manner as described above.

Agrobacterium of each of the groups was selected using a selection medium to which 50 µg/mL of kanamycin was added, and was then cultured at 28°C until the bacterial concentration became 0.8 at OD₆₀₀. The resulting Agrobacterium was suspended in an immersion buffer (10 mM morpholineprapanesulfonic acid, 10 mM MgCl₂, and 150 µM acetosyrigone, pH 5.6) at a bacterial concentration of 0.8 at OD₆₀₀. A 3-week-old tobacco *(N. benthamiana)* plant having seed leaves was immersed in the resulting Agrobacterium suspension so that the tobacco was infected with Agrobacterium. Each of the infected tobacco plants thus obtained was grown in a chamber at 25°C, and the infected tobacco plants after growth were observed (Fig. 1). The expression of the gene encoding the PAM765oy peptide in the infected plant was confirmed by RT-PCR.

As shown in Fig. 1, in the plant into which the PAM765oy peptide was introduced (B: pCAMV-TENGU), branching was significantly promoted and dwarfism was induced, as compared with the control plants (A: empty pCAMV vector, C: pCAMV-PAM486). This branch formation was similar to the branch formation characteristically caused by the infection with phytoplasmas.

Furthermore, as shown in Fig. 2, in the plant into which the PAM765oy peptide was introduced (pCAMV-TENGU), the number of leaves was significantly larger than that of the control plant (pCAMV).

Thus, the PAM765 peptide has an activity that promotes branch formation and dwarfism in plants. In addition, by simply expressing the PAM765 peptide in a part of a living plant, the effect of the expression is exerted in the entire plant.

### <EXAMPLE 2>

This example shows that PAM765 transgenic plants exhibit symptoms of promotion of branch formation, dwarfism, sterility, and multiple flower bud formation as in phytoplasma-infected wild-type plants.

### == Production of transgenic plants ==

A method for producing Tengu transgenic *A*. *thaliana* that expresses the PAM765oy peptide will be described below.

First, a DNA sequence (Seq No. 10) encoding the PAM765oy peptide was amplified by PCR using KOD DNA polymerase (Toyobo Co., Ltd.). Total DNA prepared as in Example 1 was used as a template of the PCR reaction. Oligonucleotides having the sequences below were used as primers.
Primer S2:
   cgggatcctggtcagtcccttATGGACCAAGATGATGATATTGAAAACG (sequence number 13)
Primer AS2:
   cgagctcTTAGGCATCTTTCTCGCCCTTTTGCAATAAATCTTGACA (sequence number 14)

The PCR product was cleaved with restriction enzymes (BamHI and SacI) and inserted into a multi-cloning site of a binary vector pBI121 (Clontech Laboratories Inc.) having a cauliflower mosaic virus (CaMV) 35S promoter to produce a pBI121-TENGU recombinant viral vector. Subsequently, the resulting recombinant plasmid vector (pBI121-TENGU) was introduced into Agrobacterium EHA 105 strain by an electroporation method. As a control group, a recombinant vector (pBI121-GUS) (Clontech Laboratories Inc.) containing a gene (GUS gene) encoding GUS (β-glucuronidase) protein was introduced into Agrobacterium EHA 105 strain in the same manner.

*A.* thaliana plants (ecotype, Col-0) were transformed by a floral dip method (Cough & Bent, The Plant Journal, 16: 735-743, 1996) using the resulting Agrobacterium. The transformants were selected by plating seeds of T1 plants on a kanamycin selection medium (containing salts for Murashige and Skoog medium (Wako Pure Chemical Industries, Ltd.), MS vitamin (Sigma), 1% sucrose, 0.7% agar, and 50 µg/mL kanamycin). The expression of the gene encoding the PAM765oy peptide was confirmed by RT-PCR.

In the Tengu transgenic group (N = 87) and the GUS transgenic control group (N = 25), each of the plants was observed one month after germination, and the number of plants that expressed a symptom and the number of normal plants were counted. The results of the counted numbers are shown in Table 1.

### [Table 1]

**Table 1 The proportion of transgenic A. thaliana plants that exhibited promotion of branching or dwarfism**

| The numbers in parentheses represent percentages. | | | |
|---|---|---|---|
| Transgenic | Plants that developed symptom of disease/Normal plants | Plants in which branching was promoted/Normal plants | Dwarf plants/Normal plants |
| GUS | 0/25 (0) | 0/25 (0) | 0/25 (0) |
| Tengu | 24/87 (27.6)** | 18/87 (20.7) | 6/87 (6.9)* |

| | | | |
|---|---|---|---|
| *P < 0.01, **P < 0.001 | | | |

As is apparent from Table 1, no plant that developed a symptom was observed in the GUS transgenic control group. In contrast, in the Tengu transgenic group, 27.6% of the plants developed a symptom, and thus the proportion of promotion of branching and/or dwarfism was significantly higher (Fisher's exact probability test).

### == Infection of wild-type plants with phytoplasma via leafhopper ==

*A.* thaliana plants (ecotype, Col-0) were grown in a chamber maintained at 25°C. Plants at the growth stage of 4 to 5 rosette leaves were covered with clear tubes. Five OY phytoplasma-infected leafhoppers *(M. striifrons)* were released into each of the tubes covering the plants for 5 days so that the wild-type A. thaliana was infected with a phytoplasma.

Fig. 3 shows the results in which examples of the Tengu transgenic plants that developed the symptoms shown in Table 1 are compared with examples of plants infected with a phytoplasma via leafhoppers, a non-infected plant, and a GUS transgenic plant.

In the plants infected with a phytoplasma via leafhoppers (B and C), severe dwarfism and branching were observed compared with the non-infected wild-type plant (A). On the other hand, the Tengu transgenic plants (pBI121-TENGU) also exhibited morphologies similar to those of the phytoplasma-infected plants, and plants in which the internodes of the stems are shortened, which developed dwarfism, and which produced sterile flowers (E) and plants that exhibited significant branch formation (F) were observed. The GUS transgenic plants (D), a control group, showed no symptom like the non-infected plant (A).

Furthermore, as shown in Fig. 4, in plants infected with a phytoplasma via leafhoppers (G to I) and the Tengu transgenic plants (B to E), a symptom in which a plurality of branches having a flower bud grew from a single node (B to D), and a symptom in which a plurality of flower buds grew from a single node (D) were exhibited, and sterile flowers (having no reproductive organ) (E) were also observed. In contrast, in the GUS transgenic plants (A) and the non-infected wild-type plants (F), which were control groups, these symptoms were not observed.

As described above, the PAM765 peptide promoted branch formation, dwarfism, reproductive organ formation failure (sterility), and multiple flower bud formation, all of which are caused by phytoplasmas.

### Industrial Applicability

According to the present invention, it is possible to provide a method for promoting branch formation and/or inducing dwarfism and/or causing sterility of plants without mediating infection with a non-recombinant phytoplasma, and an agent for promoting branch formation and/or inducing dwarfism and/or causing sterility and/or promoting multiple flower bud formation of plants without mediating infection with a non-recombinant phytoplasma.

## Claims

1. A method for promoting branch formation, and/or inducing dwarfism, and/or causing sterility, and/or promoting multiple flower bud formation of a plant, the method comprising:
introducing a PAM765 peptide into the plant without infection by a non-recombinant phytoplasma.

2. The method according to Claim 1, wherein the PAM765 peptide has any one of amino-acid sequences represented by Seq No. 1 to 3.

3. The method according to Claim 1, wherein
the PAM765 peptide has an amino-acid sequence in which several amino-acid residues are deleted, inserted, or substituted in any one of amino-acid sequences represented by Seq No. 1 to 3, and can promote branch formation, and/or induce dwarfism, and/or cause sterility, and/or promote multiple flower bud formation, of a plant.

4. The method according to any one of Claims 1 to 3,
wherein
DNA encoding the PAM765 peptide is expressed in the plant.

5. The method according to Claim 4, wherein
a transgenic plant is produced using the DNA encoding the PAM765 peptide.

6. An agricultural-chemical composition for promoting branch formation, and/or inducing dwarfism, and/or causing sterility, and/or promoting multiple flower bud formation, of a plant, comprising a PAM765 peptide or a gene encoding the PAM765 peptide.

7. An agent for promoting branch formation, and/or inducing dwarfism, and/or causing sterility, and/or promoting multiple flower bud formation, of a plant, comprising a PAM765 peptide or a gene encoding the PAM765 peptide.


SEQUENCE LISTING

<110>  The University of Tokyo

<120>  Method of promoting plant witches' broom symptom and/or dwarfism
        and/or sterilization and/or multiple flower formation

<130>  ICC00106EP

<160>  14

<170>  PatentIn version 3.5

<210>  1
<211>  38
<212>  PRT
<213>  Artificial Sequence

<220>
<223>  including undetermined amino acids


<220>
<221>  MISC_FEATURE
<222>  (13)..(13)
<223>  Xaa can be any naturally occurring amino acids

<220>
<221>  MISC_FEATURE
<222>  (22)..(22)
<223>  Xaa can be any naturally occurring amino acids

<220>
<221>  MISC_FEATURE
<222>  (25)..(25)
<223>  Xaa can be any naturally occurring amino acids

<400>  1

Asp Gln Asp Asp Asp Ile Glu Asn Val Ile Thr Leu Xaa Glu Thr Lys
1               5                   10                  15


Glu Asn Gln Thr Glu Xaa Ile Lys Xaa Gln Cys Gln Asp Leu Leu Gln
            20                  25                  30


Lys Gly Glu Lys Asp Ala
        35


<210>  2
<211>  38
<212>  PRT
<213>  Phytoplasma asteris

<400>  2

Asp Gln Asp Asp Asp Ile Glu Asn Val Ile Thr Leu Ile Glu Thr Lys
1               5                   10                  15

Glu Asn Gln Thr Glu Gln Ile Lys Ile Gln Cys Gln Asp Leu Leu Gln
            20                  25                  30

Lys Gly Glu Lys Asp Ala
        35

<210>  3
<211>  38
<212>  PRT
<213>  Phytoplasma asteris

<400>  3

Asp Gln Asp Asp Asp Ile Glu Asn Val Ile Thr Leu Thr Glu Thr Lys
1               5                   10                  15

Glu Asn Gln Thr Glu Glu Ile Lys Met Gln Cys Gln Asp Leu Leu Gln
            20                  25                  30

Lys Gly Glu Lys Asp Ala
        35

<210>  4
<211>  70
<212>  PRT
<213>  Artificial Sequence

<220>
<223>  including undetermined amino acids

<220>
<221>  MISC_FEATURE
<222>  (5)..(5)
<223>  Xaa can be any naturally occurring amino acids

<220>
<221>  MISC_FEATURE
<222>  (7)..(7)
<223>  Xaa can be any naturally occurring amino acids

<220>
<221>  MISC_FEATURE
<222>  (9)..(9)
<223>  Xaa can be any naturally occurring amino acids

<220>
<221>  MISC_FEATURE
<222>  (45)..(45)
<223>  Xaa can be any naturally occurring amino acids

<220>
<221>  MISC_FEATURE
<222>  (54)..(54)
<223>  Xaa can be any naturally occurring amino acids

<220>
<221>  MISC_FEATURE
<222>  (57)..(57)
<223>  Xaa can be any naturally occurring amino acids

<400>  4

Met Val Lys Leu Xaa Lys Xaa Lys Xaa Lys Leu Leu Ile Phe Ala Gly
1               5                   10                  15

Phe Trp Ala Ile Leu Leu Phe Leu Asn His Asn Tyr Leu Ile Phe Ala
            20                  25                  30

Asp Gln Asp Asp Asp Ile Glu Asn Val Ile Thr Leu Xaa Glu Thr Lys
        35                  40                  45

Glu Asn Gln Thr Glu Xaa Ile Lys Xaa Gln Cys Gln Asp Leu Leu Gln
    50                  55                  60

Lys Gly Glu Lys Asp Ala
65                  70

<210>  5
<211>  70
<212>  PRT
<213>  Phytoplasma asteris

<400>  5

Met Val Lys Leu Gln Lys Asp Lys Val Lys Leu Leu Ile Phe Ala Gly
1               5                   10                  15

Phe Trp Ala Ile Leu Leu Phe Leu Asn His Asn Tyr Leu Ile Phe Ala
            20                  25                  30

Asp Gln Asp Asp Asp Ile Glu Asn Val Ile Thr Leu Thr Glu Thr Lys
        35                  40                  45

Glu Asn Gln Thr Glu Glu Ile Lys Met Gln Cys Gln Asp Leu Leu Gln
    50                  55                  60

Lys Gly Glu Lys Asp Ala
65                  70

<210>  6
<211>  70
<212>  PRT
<213>  Phytoplasma asteris

<400>  6

Met Val Lys Leu Gln Lys Asp Lys Val Lys Leu Leu Ile Phe Ala Gly
1               5                   10                  15

Phe Trp Ala Ile Leu Leu Phe Leu Asn His Asn Tyr Leu Ile Phe Ala
            20                  25                  30

Asp Gln Asp Asp Asp Ile Glu Asn Val Ile Thr Leu Thr Glu Thr Lys
        35                  40                  45

Glu Asn Gln Thr Glu Glu Ile Lys Met Gln Cys Gln Asp Leu Leu Gln
    50                  55                  60

Lys Gly Glu Lys Asp Ala
65                  70

<210>  7
<211>  39
<212>  PRT
<213>  Artificial Sequence

<220>
<223>  including undetermined amino acids, Met artificially added to N
       terminal

<220>
<221>  MISC_FEATURE
<222>  (1)..(1)
<223>  artificially added Met

<220>
<221>  MISC_FEATURE
<222>  (14)..(14)
<223>  Xaa can be any naturally occurring amino acids

<220>
<221>  MISC_FEATURE
<222>  (23)..(23)
<223>  Xaa can be any naturally occurring amino acids

<220>
<221>  MISC_FEATURE
<222>  (26)..(26)
<223>  Xaa can be any naturally occurring amino acids

<400>  7

Met Asp Gln Asp Asp Asp Ile Glu Asn Val Ile Thr Leu Xaa Glu Thr
1               5                   10                  15

Lys Glu Asn Gln Thr Glu Xaa Ile Lys Xaa Gln Cys Gln Asp Leu Leu
            20                  25                  30

Gln Lys Gly Glu Lys Asp Ala
        35

<210>  8
<211>  39
<212>  PRT
<213>  Artificial Sequence

<220>
<223>  Met artificially added to N terminal

<220>
<221>  MISC_FEATURE
<222>  (1)..(1)
<223>  artificially added Met

<400>  8

Met Asp Gln Asp Asp Asp Ile Glu Asn Val Ile Thr Leu Ile Glu Thr
1               5                   10                  15

Lys Glu Asn Gln Thr Glu Gln Ile Lys Ile Gln Cys Gln Asp Leu Leu
            20                  25                  30

Gln Lys Gly Glu Lys Asp Ala
        35

<210>  9
<211>  39
<212>  PRT
<213>  Artificial Sequence

<220>
<223>  Met artificially added to N terminal

<220>
<221>  MISC_FEATURE
<222>  (1)..(1)
<223>  artificially added Met

<400>  9

Met Asp Gln Asp Asp Asp Ile Glu Asn Val Ile Thr Leu Thr Glu Thr
1               5                   10                  15

Lys Glu Asn Gln Thr Glu Glu Ile Lys Met Gln Cys Gln Asp Leu Leu
            20                  25                  30

Gln Lys Gly Glu Lys Asp Ala
        35

<210>  10

<211>  117
<212>  DNA
<213>  Artificial Sequence

<220>
<223>  atg (start codon) artificially added


<220>
<221>  CDS
<222>  (1)..(3)
<223>  artificially added atg (start codon)

<400>  10
atg gaccaagatg atgatattga aaacgtgata actctaattg aaactaaaga          53
Met
1

gaatcaaaca gaacaaataa aaatacaatg tcaagattta ttgcaaaagg gcgagaaaga    113

tgcc                                                                 117


<210>  11
<211>  47
<212>  DNA
<213>  Artificial Sequence

<220>
<223>  Primer

<400>  11
acgcgtcgac atggaccaag atgatgatat tgaaaacgtg ataactc                  47


<210>  12
<211>  48
<212>  DNA
<213>  Artificial Sequence

<220>
<223>  Primer

<400>  12
tgacccgggt taggcatctt tctcgccctt ttgcaataaa tcttgaca                 48


<210>  13
<211>  49
<212>  DNA
<213>  Artificial Sequence

<220>
<223>  Primer

<400>  13
cgggatcctg gtcagtccct tatggaccaa gatgatgata ttgaaaacg                49


<210>  14
<211>  46

<212>  DNA
<213>  Artificial Sequence

<220>
<223>  Primer

<400>  14
cgagctctta ggcatctttc tcgccctttt gcaataaatc ttgaca                    46